(19) Europäisches Patentamt
European Patent Office
Office européen des brevets

(11) Veröffentlichungsnummer: **0 083 427**
**B1**

(12) # EUROPÄISCHE PATENTSCHRIFT

(45) Veröffentlichungstag der Patentschrift: **10.10.84**

(51) Int. Cl.³: **C 07 C 45/78,** C 07 C 45/38, C 07 C 47/04

(21) Anmeldenummer: **82111520.1**

(22) Anmeldetag: **11.12.82**

(54) **Verfahren zur Herstellung von konzentrierten, wässrigen Lösungen von Formaldehyd und Harnstoff.**

(30) Priorität: **31.12.81 DE 3152005**

(43) Veröffentlichungstag der Anmeldung: **13.07.83 Patentblatt 83/28**

(45) Bekanntmachung des Hinweises auf die Patenterteilung: **10.10.84 Patentblatt 84/41**

(84) Benannte Vertragsstaaten: **BE CH DE FR GB IT LI**

(56) Entgegenhaltungen:
**EP - A - 0 017 206**
**FR - A - 1 374 371**
**FR - A - 2 289 537**

(73) Patentinhaber: **BASF Aktiengesellschaft, Carl-Bosch-Strasse 38, D-6700 Ludwigshafen (DE)**

(72) Erfinder: **Aicher, Albrecht, Sonnenstrasse 21, D-6710 Frankenthal (DE)**
Erfinder: **Petri, Norbert, Dr., Max-Beckmann-Strasse 17, D-6710 Frankenthal (DE)**
Erfinder: **Reuss, Guenter, Dr., Theaterplatz 10, D-6700 Ludwigshafen (DE)**
Erfinder: **Sebastian, Robert, Katharinenstrasse 4, D-6705 Deidesheim (DE)**

EP 0 083 427 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von konzentrierten, wäßrigen Lösungen von Formaldehyd und Harnstoff durch oxidierende Dehydrierung von Methanol mit Luft in Gegenwart eines Silberkatalysators bei erhöhter Temperatur, anschließender Absorption des gebildeten Formaldehyds in wäßriger Formaldehyd-Harnstofflösung mit einem Molverhältnis von 2 bis 5 Mol Formaldehyd je 1 Mol Harnstoff bei pH von 4 bis 7, anschließender Absorption des restlichen Formaldehyds in einer Lösung von Harnstoff und Formaldehyd in einem Molverhältnis von 1,8 bis 2,6 Mol Formaldehyd je Mol Harnstoff bei pH 6,5 bis 8 und schließlich in einer Formaldehyd-Harnstoff-Lösung mit einem Molverhältnis von 0,03 bis 0,5 Mol Formaldehyd je 1 Mol Harnstoff bei pH 8 bis 9, wobei gleichzeitig ein Anteil der Lösung aus der 1. Stufe mit einem Anteil der Lösung aus der 3. Stufe miteinander so gemischt werden, daß sich in dem gebildeten Gemisch ein Molverhältnis kleiner als 1,8 Mol Formaldehyd je Mol Harnstoff einstellt, und das Gemisch der Lösung der Stufe 2 zugeführt und ein weiterer Anteil der Lösung aus Stufe 2 der Stufe 1 zugeführt und ein weiterer Anteil der Lösung aus Stufe 2 mit Säure bei pH 4 bis 6,5 behandelt wird und in die Stufe 2 zurückkehrt, und man gewünschtenfalls die aus der 1. Stufe abgezogene Lösung des Endstoffs einer Luftwäsche unterzieht.

Bei der technischen Herstellung von Formaldehyd aus Methanol durch dehydrierende Oxidation mit Luft an Silberkatalysatoren in Gegenwart von Wasserdampf wird üblicherweise der Formaldehyd aus den Reaktionsgasen mit Wasser ausgewaschen. Wenn der Formaldehyd, wie es in großem Ausmaße geschieht, zur Herstellung von Polykondensationsharzen, z. B. von Harnstoff-Formaldehydharzen verwendet wird, müssen derartige Harzlösungen eingeengt werden, wobei erhebliche Mengen Wasser zu verdampfen sind.

Um das Einengen der Harzlösungen, das zeitraubend ist, einen großen Energieaufwand verlangt und mit Formaldehydverlusten verbunden ist, zu vermeiden, hat man schon versucht, hochkonzentrierte Formaldehydlösung durch Absorption der Formaldehydgase in sauren oder basischen Harnstofflösungen herzustellen. Sowohl das saure als auch das basische Verfahren haben gewisse Nachteile. So scheidet sich bei der Absorption des gasförmigen Formaldehyds im sauren Bereich, insbesondere bei Abwesenheit von restlichem Methanol, leicht unlöslicher Methylenharnstoff ab. Im alkalischen Bereich wiederum scheiden sich leicht unlösliche Methylolharnstoffe ab, die zur Verstopfung der Absorptionstürme führen können.

Einen besseren Weg zeigen Verfahren, bei denen die Absorption des Formaldehyds in Lösungen von Formaldehyd und Harnstoff, wo beide Komponenten schon bis zu einem gewissen Grad miteinander reagiert haben (Ankondensation), z. B. in Lösungen entsprechender Vorkondensate, erfolgt. Als Vorkondensate bezeichnet man in diesem Zusammenhang niedermolekulare Verbindungen von Harnstoff und Formaldehyd, in der Hauptsache ein Gemisch von Methylolharnstoff, Dimethylolharnstoff, Trimethylolharnstoff und Tetramethylolharnstoff und daneben Methylendiharnstoff, Dimethylentriharnstoff, Trimethylentetraharnstoff; ebenfalls sind in solchen Vorkondensatgemischen auch unsubstituierter Harnstoff, Formaldehyd, niedermolekulare Polymethylolverbindungen der vorgenannten Methylenharnstoffe, Methylenetherverbindungen mit Brückenglieder $-NH-CH_2-O-CH_2-NH$ oder verzweigte oder vernetzte, niedermolekulare Methylenharnstoffe, z. B. mit der Gruppe

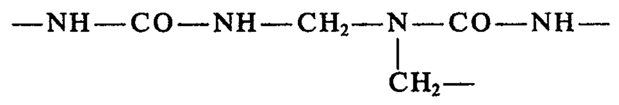

vorhanden.

Es ist aus der deutschen Patentschrift 1 168 882 bekannt, Formaldehyd aus dem durch dehydrierende Oxidation von Methylalkohol in Gegenwart eines Silberkatalysators stammenden Gas zu absorbieren, indem man als Absorptionsflüssigkeit wäßrige Lösungen von Harnstoff-Formaldehyd-Vorkondensaten mit einem Molverhältnis von Harnstoff zu Formaldehyd von 1 : 1,8 bis etwa 1 : 3 verwendet und während der Absorption bei erhöhter Temperatur einen pH-Wert von 2,5 bis 7 aufrechterhält. Nur ein Teil des in die Lösung eintretenden Formaldehyds wird absorbiert, der Rest entweicht während der Absorption wieder in das Dampf/Gas-Gemisch und gelangt so in das Abgas; wie auch Beispiel 1 zeigt, können auf diese Weise nur Lösungen mit bis zu 40 bis 41 Gew.-% Formaldehyd erhalten werden.

Aus der britischen Patentschrift 1 235 138 ist es bekannt, Formaldehyd durch Umsetzung von Methanol in der Gasphase mit Sauerstoff enthaltenden Gasen bei Temperaturen von 600 bis 800°C an Silberkatalysatoren herzustellen und das Formaldehyd enthaltende Gas in mindestens 3 Stufen bei 60 bis 90°C zu absorbieren, wobei man das Gas in der ersten bis zur vorletzten Stufe mit wäßrigen, sauren Lösungen von Harnstoff-Formaldehyd-Vorkondensaten und in der letzten Stufe mit einer wäßrigen Harnstofflösung in Berührung bringt.

Bei allen diesen Silber als Katalysator verwendenden Verfahren fallen im allgemeinen Lösungen von Formaldehyd und Harnstoff an, die 35 bis 42 Gew.-% Formaldehyd enthalten. Für die Weiterverarbeitung sind aber Lösungen mit einem größeren Formaldehydanteil erwünscht. Die bei diesen Verfahren übliche Arbeitsweise, durch Einengen der Absorptionslösungen hochkonzentrierte Lösungen zu erhalten, ist im Hinblick auf Wirtschaftlichkeit und einfache Betriebsweise unbefriedigend.

Es ist aus der deutschen Offenlegungsschrift 2 224 258 bekannt, die Absorption des gebildeten Formaldehyds in einer wäßrigen Lösung von Formaldehyd und Harnstoff bei einem pH unterhalb 7 durchzuführen und die so erhaltenen Lösungen mit Luft, die anschließend für die Dehydrierung verwendet wird, zu behandeln.

In einer bevorzugten Ausführungsform läßt man das gasförmige Reaktionsgemisch nacheinander mindestens 3 Absorptionskolonnen durchlaufen, wobei man es in der ersten bis zur vorletzten Stufe mit wäßrigen Lösungen von Harnstoff-Formaldehyd-Vorkondensaten und in der letzten Stufe miteiner wäßrigen Harnstofflösung in Berührung bringt. Vorzugsweise verwendet man insgesamt 4 oder allenfalls 5 hintereinandergeschaltete Absorptionsstufen. In der vorletzten bis zur ersten Absorptionsstufe wird als Absorptionsflüssigkeit eine wäßrige Lösung eines Harnstoff-Formaldehyd-Vorkondensates verwendet, welches aus einer wäßrigen Harnstoff-Formaldehyd-Lösung mit einem Molverhältnis Formaldehyd zu Harnstoff von mindestens 1,8 bis etwa 2,8, vorzugsweise von 2 bis 2,4 zu 1, durch Kondensation im sauren Bereich, beispielsweise unter Zugabe von Ameisensäure, bei einem pH-Wert von vorzugsweise 4 bis 6,5, insbesondere 5 bis 6, und einer Temperatur von 60 bis 95°C, vorzugsweise von 85 bis 95°C, in an sich bekannter Weise hergestellt wird. Die Kondensation wird dabei so weit geführt, daß in den Lösungen etwa 7 bis 25 Gew.-% des Formaldehyds in Form von Methylenbrücken und 30 bis 60 Gew.-% in Form von Methylolharnstoffen gebunden sind. In den Lösungen wird anschließend durch weitgehende Neutralisation, z. B. durch Zugabe von Natronlauge, ein pH-Wert von unterhalb 7, zweckmäßig 6 bis 7, eingestellt. Das Molverhältnis Formaldehyd (freier und kondensierter) zu Harnstoff in den aus den einzelnen Absorptionsstufen abgezogenen Lösungen beträgt bei der 4. Stufe in der Regel etwa 0,1 bis 0,4, bei der 3. Stufe gewöhnlich etwa 2 bis 2,8, übersteigt bei der 2. Stufe im allgemeinen nicht 3, und erreicht bei der 1. Stufe im allgemeinen Werte von 3,5 bis 6. Die aus der ersten bis vorletzten Stufe abgezogenen Lösungen haben im allgemeinen pH-Werte zwischen 4 und 7, vorzugsweise 5 bis 6 in der 1., 5,5 bis 6,5 in der 2., 6 bis 6,9 in der 3., 7,5 bis 9 in der 4. Stufe.

Die bevorzugte und im Ausführungsbeispiel veranschaulichte Ausführungsform des Verfahrens besteht darin, daß man die aus der letzten Absorptionsstufe erhaltene wäßrige Formaldehyd-Harnstoff-Lösung zur Herstellung der wäßrigen Lösung des Formaldehyd-Harnstoff-Vorkondensates verwendet. Man arbeitet so, daß man die aus der letzten Stufe erhaltene Lösung mit soviel der aus der ersten Absorptionsstufe abgezogenen konzentrierten Formaldehydlösung mischt, daß die Mischung je Mol Harnstoff 1,8 bis etwa 2,8, vorzugsweise 2 bis 2,4 Mol Formaldehyd enthält. Dabei ist es wichtig, den Mischvorgang innerhalb sehr kurzer Zeit, z. B. innerhalb von 1 bis 5 Sekunden auszuführen, um die Ausscheidung von festen Kondensaten zu vermeiden. Die Mischung wird anschließend kondensiert, neutralisiert und schließlich in der letzten Stufe vorgeschalteten Absorptionsstufen als Absorptionsflüssigkeit bei einem pH unterhalb 7 verwendet. Lösungen von im allgemeinen 47 bis 56 Gew.-% absorbiertes Formaldehyd werden erhalten.

Die DE-AS 2 451 990 lehrt, daß man die Absorption in einer ersten Stufe in einer wäßrigen Lösung von Formaldehyd und Harnstoff mit einem Molverhältnis von 2,3 bis 5 Mol Formaldehyd je 1 Mol Harnstoff, bei einem pH zwischen 5 und 7, dann in einer 2. Stufe mit einer wäßrigen Lösung von 25 bis 32 Gew.-% Harnstoff, von 4 bis 10 Gew.-% freiem Formaldehyd und von 15 bis 25 Gew.-% Methylolharnstoffverbindungen bei einem pH von 7 bis 8 und in einer 3. Stufe in einer wäßrigen Lösung von Formaldehyd und Harnstoff mit einem Molverhältnis von 0,05 bis 0,4 Mol Formaldehyd je 1 Mol Harnstoff bei einem pH von 8 bis 9 durchgeführt wird.

Es wurde nun gefunden, daß man konzentrierte, wäßrige Lösungen von Formaldehyd und Harnstoff durch oxidierende Dehydrierung von Methanol mit Luft in Gegenwart eines Silberkatalysators bei erhöhter Temperatur und anschließender Absorption des so gebildeten Formaldehyds in einer wäßrigen Lösung von Formaldehyd und Harnstoff vorteilhaft herstellt, wenn die Absorption in einer ersten Stufe in einer wäßrigen Lösung von Formaldehyd und Harnstoff mit einem Molverhältnis von 2 bis 5 Mol Formaldehyd je 1 Mol Harnstoff bei einem pH von 4 bis 7, dann in einer 2. Stufe mit einer wäßrigen Lösung von Formaldehyd und Harnstoff in einem Molverhältnis von 1,8 bis 2,6 Mol Formaldehyd je Mol Harnstoff bei einem pH von 6,5 bis 8 und in einer 3. Stufe in einer wäßrigen Lösung von Formaldehyd und Harnstoff mit einem Molverhältnis von 0,03 bis 0,5 Mol Formaldehyd je 1 Mol Harnstoff bei einem pH von 8 bis 9 durchgeführt wird, wobei gleichzeitig ein Anteil der Lösung aus der 1. Stufe mit einem Anteil der Lösung aus der 3. Stufe miteinander so gemischt werden, daß sich in dem gebildeten Gemisch ein Molverhältnis kleiner als 1,8 Mol Formaldehyd je Mol Harnstoff einstellt und das Gemisch der Lösung der Stufe 2 zugeführt und ein weiterer Anteil der Lösung aus Stufe 2 der Stufe 1 zugeführt und ein weiterer Anteil der Lösung aus Stufe 2 mit Säure bei pH 4 bis 6,5 behandelt wird und in die Stufe 2 zurückkehrt, und man gewünschtenfalls die aus der 1. Stufe abgezogene Lösung des Endstoffs einer Luftwäsche unterzieht.

Die Erfindung geht von der Beobachtung aus, daß nur eine Kombination ausgewählter Bedingungen in der Absorption, insbesondere eine dreistufige Arbeitsweise mit einem sauren, einem schwach sauren bis schwach alkalischen und einen alkalischen Absorptionsmedium und einer speziellen Zusammensetzung der 3 Medien sowie auch insbesondere des Lösungsgemischs der Stufen 1 und 3 und der säurebehandelten Lösung mit Bezug auf die Menge an Harnstoff, freiem Formaldehyd und Methylolharnstoffverbindungen sowie Nebenstoffen die Herstellung hochkonzentrierter, stabiler, wäßriger Lösungen erzielt. Im Vergleich zu den erstgenannten bekannten Verfahren liefert das Verfahren nach

der Erfindung auf einfacherem und wirtschaftlicherem Wege hochkonzentrierte, wäßrige Lösungen von Formaldehyd und Harnstoff. Lösungen von im allgemeinen 35 bis 45 Gew.-% absorbiertem Formaldehyd werden erhalten, unter Verwendung der Luftwäsche 45 bis 56 Gew.-% absorbiertem Formaldehyd.

Im Vergleich zu dem in der deutschen Offenlegungsschrift 2 224 258 beschriebenen Verfahren wird die gesonderte Herstellung der Vorkondensate und damit zusätzliche Umsetzungs- und Neutralisationsoperationen eingespart. Die Formaldehyd-Harnstoff-Lösungen sind salzärmer und können daher für zahlreiche Synthesen, z. B. bei der Leimherstellung ohne Einengung der Leime, direkt weiterverarbeitet werden und ergeben reinere Folgeprodukte. Die Menge an Methylenharnstoffen und Polymethylolverbindungen von Methylenharnstoffen sowie höhermolekularen Kondensaten, die die Weiterverarbeitung zu Klebmitteln stören, sind geringer. Der Gehalt an erwünschten Methylolharnstoffen ist höher. Daß ein Molverhältnis von kleiner als 1,8 $CH_2O$ : 1 Harnstoff als Mischung der 1. und 3. Stufe eine wesentliche, vorteilhafte Rolle spielt, konnte nicht erwartet werden. Diese vorteilhaften Ergebnisse sind im Hinblick auf den Stand der Technik überraschend, da ja die Absorption sowohl im sauren (Stufe 1) als auch im basischen Medium (Stufen 2, 3) stattfindet und man eher eine Erhöhung der Menge an Methylenkondensaten und Abscheidung unlöslicher Methylolharnstoffe hätte erwarten müssen.

Die in DE-PS 1 168 882 und DE-PS 2 451 990 beschriebenen Verfahren sind unelastisch, wenn man damit ein trübungsfreies Produkt längerer Lebensdauer und hohen reaktiven Methylolanteils herstellen will. Das erstgenannte Verfahren ist besonders empfindlich gegen Änderungen im Absorptionsgeschehen (Temperatur, Druck, Umsatz) und liefert bei Molverhältnisschwankungen in den Absorptionsstufen leicht ein zu hoch oder zu niedrig kondensiertes, in beiden Fällen unbefriedigendes Produkt, das oft trüb ist oder zur Nachkristallisation neigt. Auch nach dem zweiten Verfahren ist letztlich die gesamte Absorptionslösung innerhalb der Stufe 1 einschließlich Zulauf aus den hinteren Stufen im unteren pH-Bereich und bei hohen Temperaturen einer unkontrollierten Kondensation ausgesetzt, so daß auch so erhaltene Produkte keinen hohen Qualitätsanforderungen genügen. Man erreicht im Vergleich zum erfindungsgemäßen Verfahren nur höhere Methylen-Gehalte (c—f der Tabelle) in den Lösungen und niedrigere Methylolanteile (b der Tabelle), die Produkte neigen jedoch zu nachträglicher Trübung und Kristallisation.

Bei dem in der britischen Patentschrift 1 235 138 beschriebenen Verfahren ist nachteilig, daß man mit den Absorptionslösungen der beanspruchten Molverhältnisse (1,8 bis 2,8 Mol Formaldehyd pro Mol Harnstoff) nur dann zu stabilen Formaldehyd-Harnstoff-Lösungen gelangt, wenn sich während der Kondensation relativ viel, z. B. 15 bis 40Gew.-% des Formaldehyds, in Form von Methylenbrücken bilden. Solche Formaldehyd-Harnstoff-Lösungen sind aber für die Verarbeitung zu Kondensationsharzen des heutigen Qualitätsstandes nahezu ungeeignet.

Bei der Säurebehandlung von Anteilen der Stufe 2 des erfindungsgemäßen Verfahrens kann z. B. durch Veränderung der Teilstrom-Menge unabhängig die Verweilzeit beeinflußt und ohne zusätzliche Puffer-Zugabe der gewünschte Kondensationsgrad eingestellt werden. Damit gelingt es, den Puffersalz-Gehalt im Endstoff zu vermindern. Der apparative Aufwand ist wesentlich kleiner, weil nur eine Teilmenge der gesamten Absorptionslösung kondensiert werden braucht. Dies ergibt auch eine Einsparung von Energie, da Kondensationswärme wesentlich geringer als Methylolierungswärme ist. Es ergibt sich als weiterer Vorteil besseres Absorptionsverhalten der so hergestellten Lösungen, da weniger freier Formaldehyd vorhanden.

In folgender Tabelle werden die Analysenergebnisse der endgültigen Absorptionslösungen (Lösung des Endstoffs), die die vorgenannten Verfahren liefern, aufgeführt. Unter Gesamtharnstoff bzw. Gesamtformaldehyd werden die Gesamtmengen an Harnstoff bzw. Formaldehyd (freier und gebundener), verstanden. Die erfindungsgemäßen Werte in Zeilen a—f sind in Gewichtsprozenten, bezogen auf die Gewichtsmenge Gesamtformaldehyd, angegeben.

Tabelle

| Endstoff (in Gew.-% der Lösung) | DE-PS 1 168 882 (Beispiel 1) | DE-OS 2 224 258 Spalte 6, Zeilen 31—49 (Vorzugsbereich) | DE-AS 2 451 990 (Beispiel) | Erfindungsgemäß (Seite 19) |
|---|---|---|---|---|
| Gesamtformaldehyd | 51 | 39—40 | 50 | 35—45 |
| (davon Formaldehyd in | | | | |
| a) freier Form | 40 (a+b) | 17—22 | 22,9 | 16—20 |
| b) Mono- und Polymethylolharnstoffen | | 13—17 | 22,6 | 17—21 |
| c) Mono- und Polymethylenharnstoffen | 11 (c–f) | 3—4 | 3,5 | 1,6—2,8 |
| d) Polymethylolverbindungen von Methylenharnstoffen | | 1—2 | 0,7 | 0,29—0,7 |
| e) Methylenetherverbindungen | | 0,2—0,5 | 0,2 | 0,1—0,3 |
| f) höhermolekularen Kondensaten) | | 0,2—0,4 | 0,1 | 0,01—0,2 |
| Gesamtharnstoff | 25,5 | 18—22 | 25 | 18—30 |
| Methanol | | 0,8—1,5 | 0,5 | 0,2—3 |
| Ameisensäure | | 0,01—0,05 | 0,008 | 0,005—0,05 |
| Molverhältnis ($CH_2O$ : Harnstoff) | 4 | 3,8—4,2 | 4 | 2—5 |
| (in Gew.-% des Gesamtformaldehyds) | | | | |
| Formaldehyd in freier Form (a) | 78,4 (a+b) | 43,6—55 | 45,8 | 45,7—44,5 |
| Formaldehyd in Methylolharnstoffen (b) | | 33,3—42,5 | 45,2 | 48,6—46,6 |
| Formaldehyd in Methylenverbindungen (c, d, e, f) | 21,6 | 11,3—17,3 | 9,0 | 5,7—8,9 |

Man ersieht daraus, daß bei einem Vergleich der Anteile der verschiedenen Formaldehyd enthaltenden Stoffe (a—f) am Gesamtformaldehyd der Formaldehyd in den Methylolharnstoffen (b) beim Verfahren nach der Erfindung den größten Anteil und der Formaldehyd in der Summe der Methylenverbindungen (c bis f) den kleinsten Anteil von allen betrachteten Verfahren zeigt. Solche Lösungen besitzen eine weit höhere Reaktivität bei der Weiterverarbeitung zu Kondensationsharzen und sind deshalb besser hierfür als vergleichsweise Lösungen mit geringen Methylol- und hohen Methylenwerten geeignet.

Für die Formaldehydherstellung geeignete Ausgangsstoffe sind reines Methanol, technisches Methanol oder vorteilhaft deren Mischungen mit Wasser. Auch Rohmethanol, das nach den in DE-AS 1 277 834, DP 1 136 318 und DP 1 235 881 beschriebenen Verfahren durch Abtrennung einer niedriger siedenden Fraktion, durch Behandlung mit Oxidationsmitteln und/oder Alkalien gereinigt werden kann, kann verwendet werden. Im allgemeinen wird das Methanol in Dampfform und gegebenenfalls im Gemisch mit Inertgas dem Reaktionsraum zugeführt. Man kann Methanoldampf und Wasserdampf getrennt voneinander erzeugen und mit Luft mischen, vorteilhaft wird man aber eine entsprechende wäßrige Lösung von Methanol verdampfen und Luft mit dem Dampfgemisch mischen.

Als oxidierendes Agens für die Formaldehydherstellung dient Luft. Luft und Methanol werden zweckmäßig im Molverhältnis von 1 Mol Methanol zu 0,15 bis 0,5 Mol Sauerstoff in Gestalt von Luft angewandt.

Für das Verfahren nach der Erfindung kommen die für die Herstellung von Formaldehyd im allgemeinen verwendeten Silberkatalysatoren in Betracht, z. B. die in DE-AS 1 231 229 und Ullmanns Encyklopädie der technischen Chemie, Band 7, Seiten 659 ff beschriebenen. Vorzugsweise verwendet man Zwei- und Mehrschicht-Silberkatalysatoren, z. B. die in DE-AS 1 294 366 und in der DE-OS 1 903 197 aufgeführten Katalysatoren. Bevorzugte Katalysatoren sind die in der deutschen Patentschrift 2 322 757 beschriebenen. Bezüglich Herstellung des Katalysators und Durchführung der entsprechenden Umsetzung mit diesen Katalysatoren wird auf die genannten Veröffentlichungen verwiesen.

Die Oxidation wird im übrigen in bekannter Weise durchgeführt, indem man z. B. ein Dampf/Gasgemisch aus Methanoldampf, Wasserdampf, Behandlungsluft bzw. Behandlungsgas und gegebenenfalls weiteren Anteilen von Luft und/oder Inertgas in vorgenannten Mengen bei Temperaturen von etwa 550 bis 780°C, insbesondere 640 bis 750°C, durch den Silberkatalysator leitet. Es ist dabei zweckmäßig, die die Katalysatorzone verlassenden Reaktionsgase innerhalb kurzer Zeit, beispielsweise in weniger als 0,2 Sekunden, abzukühlen, z. B. auf Temperaturen von 50 bis 170°C. Das abgekühlte Gasgemisch wird dann zweckmäßig einem Absorptionsturm zugeführt. In einer bevorzugten Ausführungsform kühlt man das gasförmige Reaktionsgemisch auf 70 bis 90°C ab, wobei sich wäßrige Formaldehydlösungen von 25 bis 50 Gew.-% Formaldehyd und von 0,5 bis 2 Gew.-% Methanol bilden, und leitet den gasförmigen und flüssigen Anteil des Reaktionsgemischs in die erste Absorptionsstufe.

Die Herstellung des Formaldehyds wird im allgemeinen bei Drücken zwischen 0,5 und 2 bar, vorzugsweise zwischen 0,8 und 1,8 bar, diskontinuierlich oder vorzugsweise kontinuierlich durchgeführt.

Die Absorption des Formaldehyds kann drucklos oder unter Druck, diskontinuierlich oder zweckmäßig kontinuierlich durchgeführt werden, wobei die vorgelegten Absorptionslösungen durch Zuführung der Komponenten Formaldehyd und Harnstoff auf das gewünschte Mengenverhältnis eingestellt wird. Unter Methylolharnstoffen (b der Tabelle) werden hier Kondensate ohne Methylenbrücken wie die Mono- und Polymethylolharnstoffe Methylolharnstoff, Dimethylolharnstoff und Trimethylolharnstoff, aber keine Polymethylolverbindungen von Methylenharnstoffen (d der Tabelle) verstanden. Man absorbiert in 3 Stufen, die jeweils eine und mehr Absorptionskolonnen umfassen können. Bevorzugt ist eine Kolonne je Absorptionsstufe. Ebenfalls ist eine bevorzugte Ausführungsform, die 1. Stufe in einer und die 2. und 3. Stufe in einer zweiten Kolonne (2. Stufe unterer und 3. Stufe oberer Teil) durchzuführen. In einer bevorzugten Ausführungsform läßt man das gasförmige Reaktionsgemisch nacheinander 2 Absorptionskolonnen bei Temperaturen von 60 bis 90°C, insbesondere 70 bis 85°C, durchlaufen, wobei man es in der letzten Kolonne (oberer Teil = Stufe 3) mit einer wäßrigen Harnstofflösung, in der 2. Stufe (unterer Teil der zweiten Kolonne) mit einem wäßrigen Gemisch der Lösungen von Harnstoff, Formaldehyd und Kondensaten von Stufe 1 und 3 behandelt. Der 1. Stufe werden das Reaktionsgemisch der Formaldehydsynthese und ein Teilstrom aus dem Sumpf der 2. Stufe, zweckmäßig 2 bis 12 Gewichtsprozent des Gesamtgewichtes des Sumpfes 2 zugeführt. Die Absorption kann diskontinuierlich oder bevorzugt, drucklos oder unter Druck, durchgeführt werden.

Zweckmäßig gewinnt man aus dem aus der letzten Absorptionsstufe entweichenden Abgase Reste an wäßrigem Methanol wieder und mischt es dem Ausgangsmethanol zu und gibt gegebenenfalls der Mischung aus wiedergewonnenen wäßrigem Methanol und Ausgangsmethanol so viel eines alkalischen Mittels zu, daß die Mischung einen pH-Wert von mindestens 9, vorzugsweise 9,5 bis 12, aufweist. Diese Verfahrensvariante wird im allgemeinen kontinuierlich drucklos oder unter Druck durchgeführt. Es ist so möglich, praktisch sämtliches Methanol aus dem Synthesegas zu gewinnen und somit einen besonders hohen Umsatz des Methanols zu erzielen.

In der letzten Stufe wird als Absorptionsflüssigkeit eine zweckmäßige 50 bis 80gewichtsprozentige, insbesondere 60 bis 70gewichtsprozentige, wäßrige Harnstofflösung verwendet, von der man, bezogen auf das für die Reaktion eingesetzte Methanol, zweckmäßig 20 bis 80 Gew.-%, vorzugsweise 30 bis 60 Gew.-%, berechnet als Harnstoff, einsetzt. Die Lösung nimmt aus dem bereits verarmten Gas den

restlichen Formaldehyd auf. Die abgezogene wäßrige Formaldehyd-Harnstoff-Lösung enthält Formaldehyd und Harnstoff im Molverhältnis 0,03 : 1 bis 0,5 : 1, vorteilhaft 0,1 : 1 bis 0,3 : 1. Die Absorptionslösung enthält im allgemeinen von 50 bis 80, vorzugsweise von 55 bis 70 Gew.-% Gesamtharnstoff, von 1,15 bis 9,5, insbesondere von 3,6 bis 6,8 Gew. % Gesamtformaldehyd in Form von 0,1 bis 3,0, vorzugsweise von 0,5 bis 1,5 Gew.-% Methylolharnstoffen, von 1 bis 6, vorzugsweise von 3 bis 5 Gew.-% Mono- und Polymethylenharnstoffen, von 0,05 bis 0,5, vorzugsweise von 0,1 bis 0,3 Gew.-% freiem Formaldehyd, von 0,05 bis 1, vorzugsweise von 0,1 bis 0,5 Gew.-% Methanol, von 0,05 bis 0,5, vorzugsweise von 0,1 bis 0,3 Gew.-% Nebenstoffe (z. B. Ammoniumcarbonat).

In der 2. Stufe, vorzugsweise der vorletzten Absorptionskolonne bzw. der unteren Hälfte einer gemeinsamen Kolonne, wird als Absorptionslösung eine wäßrige Lösung von 28 bis 31, vorzugsweise von 29 bis 30,5 Gew.-% Gesamtharnstoff, von 28 bis 37, insbesondere von 29,5 bis 35 Gew.-% Gesamtformaldehyd in Form von 7 bis 9, vorzugsweise von 7,3 bis 8,5 Gew.-% freiem Formaldehyd und von 17,4 bis 20, vorzugsweise von 18 bis 19,5 Gew.-% Methylolharnstoffen verwendet. Neben diesen Stoffen können noch weitere Stoffe, die sich z. B. bei der Herstellung der Lösung gebildet haben, vorhanden sein, zweckmäßig von 3 bis 5, vorzugsweise von 3 bis 4,5 Gew.-% Formaldehyd in Form von Mono- und Polymethylenharnstoffen, von 0,5 bis 2,4, vorzugsweise von 1 bis 2,1 Gew.-% Polymethylolverbindungen von Methylenharnstoffen, von 0,1 bis 0,4, vorzugsweise von 0,2 bis 0,3 Gew.-% Methylenetherverbindungen, von 0,05 bis 0,2, vorzugsweise von 0,08 bis 0,1 Gew.-% höhermolekularen Kondensaten, von 0,2 bis 3, vorzugsweise von 0,5 bis 1,5 Gew.-% Methanol, von 0,005 bis 0,05, vorzugsweise von 0,006 bis 0,02 Gew.-% Nebenstoffe (z. B. Ameisensäure). Das Molverhältnis Formaldehyd zu Harnstoff beträgt von 1,8 bis 2,6 : 1, vorteilhaft zwischen 1,9 und 2,4 : 1.

In der 1. Stufe, vorzugsweise ersten Absorptionskolonne, dient als Absorptionslösung eine wäßrige Lösung von Formaldehyd und Harnstoff mit einem Molverhältnis von 2 bis 5, vorzugsweise 3 bis 4,5 Mol Formaldehyd je Mol Harnstoff. Die Absorptionslösung enthält im allgemeinen von 18 bis 30, vorzugsweise von 19 bis 25 Gew.-% Gesamtharnstoff, von 35 bis 45, vorzugsweise von 38 bis 43 Gew.-% Gesamtformaldehyd in Form von 16 bis 20, vorzugsweise von 17,5 bis 19,4 Gew.-% freiem Formaldehyd, von 17 bis 21, vorzugsweise von 18 bis 20 Gew.-% Methylolharnstoffen, von 1,6 bis 2,8, vorzugsweise von 2,1 bis 2,6 Gew.-% Mono- und Polymethylenharnstoffen, von 0,29 bis 0,7, vorzugsweise von 0,3 bis 0,6 Gew.-% Polymethylolverbindungen von Methylenharnstoffen, von 0,1 bis 0,3, vorzugsweise von 0,15 bis 0,25 Gew.-% Methylenetherverbindungen, von 0,01 bis 0,2, vorzugsweise von 0,01 bis 0,15 Gew.-% höhermolekularen Kondensaten, von 0,2 bis 3, vorzugsweise von 0,4 bis 1,6 Gew.-% Methanol, von 0,005 bis 0,05, vorzugsweise von 0,006 bis 0,03 Gew.-% Nebenstoffe (z. B. Ameisensäure).

In den 3 Absorptionsstufen werden so von der wäßrigen Lösung des Harnstoff-Formaldehyd-Kondensates in der 3. Stufe im allgemeinen etwa 2 bis 6 Gew.-%, in der 2. Stufe etwa 8 bis 15 Gew.-% und in der 1. Stufe etwa 80 bis 90 Gew.-% der gesamten Formaldehydmenge absorbiert. Vorteilhaft betragen in der 1. und 3. Stufe die Verweilzeiten, bezogen auf den der Absorption zugeführten Formaldehyd aus dem Reaktionsgemisch 20 bis 120 Minuten, in der 2. Stufe 10 bis 50 Minuten. Die Sumpftemperaturen betragen zweckmäßig 75° C (Stufe 1), 74° C (Stufe 2), 80° C (Stufe 3). Die pH-Werte der Lösungen sind in der 1. Stufe von 4 bis 7, vorzugsweise 5 bis 6,5, in der 2. Stufe von 6,5 bis 8, vorzugsweise von 7 bis 7,6, in der 3. Stufe von 8 bis 9, vorzugsweise von 8,2 bis 8,6. Gegebenenfalls stellt man das pH durch Zugabe von Ameisensäure bzw. Natronlauge ein.

Die Absorptionslösung der Stufe 2 wird im wesentlichen durch Vermischen von Anteilen der Lösungen der 1. und 3. Stufe hergesellt. Die Vermischung und das so gebildete Gemisch ist ein besonderes erfinderisches Merkmal. Zweckmäßig leitet man die Lösungen der 3 Stufen in ihren jeweiligen Absorptionskolonnen im oberen Teil bzw. am Kolonnenkopf ein, pumpt den sich am Kolonnenboden — im Falle der 3. Stufe vorteilhaft am Boden des oberen Kolonnenteils — bildenden Kolonnensumpf wieder im Kreislauf zum Kolonnenkopf zurück und entnimmt aus den beiden Kreisläufen der Stufen 1 und 3 jeweils Anteile, die man miteinander mischt und zur 2. Stufe — zweckmäßig zur Mitte der vorgenannten zweiteiligen Kolonne — leitet. Auch in der 2. Stufe (Kolonne bzw. unteren Kolonnenteil) wird die Absorptionslösung im Kreislauf geführt, wobei ein Anteil zur 1. Stufe — zweckmäßig zu dem Kopf der 1. Kolonne — zurückgeführt wird. Das Reaktionsgemisch der Formaldehydherstellung trifft so in der 1. Stufe (Kolonne) im Gleichstrom in den beiden anderen im Gegenstrom auf die jeweilige Absorptionslösung.

Zweckmäßig führt man aus Stufe 1 2 bis 5 Gew.-% Absorptionslösung, bezogen auf das Gesamtgewicht des Sumpfes 1 und aus Stufe 3 1 bis 4 Gew.-% Absorptionslösung, bezogen auf das Gesamtgewicht des Sumpfes 3, ab. Vorteilhaft mischt man die Lösungen in einem Verhältnis von 1,2 bis 2,1, vorzugsweise von 1,5 bis 2 Teilen Lösung der Stufe 1 zu 1 Teil Lösung der Stufe 3, um in der 2. Stufe eine Absorptionslösung der vorgenannten Zusammensetzung zu erhalten. In dem so erhaltenen Gemisch sind ein pH von 7 bis 8, insbesondere von 7,1 bis 7,6 und ein Molverhältnis von kleiner als 1,8, zweckmäßig zwischen 1,3 und 1,8, vorzugsweise zwischen 1,6 und 1,8, insbesondere von 1,6 bis 1,76 Mol Formaldehyd je Mol Harnstoff vorteilhaft und ein besonderes erfinderisches Merkmal. Zweckmäßig betragen die Verweilzeiten vom Beginn der Mischung bis zum Eintritt in Stufe 2 1 bis 60, vorzugsweise 2 bis 30 Sekunden und die Temperatur 60 bis 90° C, insbesondere 70 bis 80° C.

Ein weiteres wichtiges Merkmal ist die Behandlung eines Anteils von Stufe 2, zweckmäßig von 10 bis 80 Gew.-%, bezogen auf Gesamtabsorptionslösung der Stufe 2, mit Säure. Als Säuren können

anorganische oder organische Säuren verwendet werden, bevorzugt Ameisensäure oder Schwefelsäure. Der Anteil (Stufe 2) wird abgezogen, in einem Behandlungsgefäß (Rührbehälter, Mischrohr) mit der Säure bei einem pH von 4 bis 6,5, vorzugsweise 4,5 bis 6 und vorteilhaft mit einer Verweilzeit von 10 bis 120, vorzugsweise 20 bis 40 Minuten, bei einer Temperatur von 50 bis 90, vorzugsweise 65 bis 80°C behandelt und der Stufe 2 wieder zugeführt. Das Molverhältnis des Anteils nach der Säurebehandlung beträgt 1,8 bis 2,6, vorzugsweise 1,9 bis 2,4 Mol Formaldehyd je Mol Harnstoff.

Aus dem die letzte Absorptionsstufe verlassenden formaldehydfreien Abgas wird nicht umgesetztes Methanol zweckmäßig durch Kondensation, z. B. in einem Kühler oder durch Absorbieren in Wasser, z. B. mit Hilfe eines Waschturms, abgetrennt. Das dabei erhaltene wäßrige Methanol wird dem Ausgangsmethanol zugeführt. So erhält man z. B. durch Abkühlen der Reaktionsgase auf 25°C eine 1- bis 4gewichtsprozentige wäßrige Methanollösung. Im allgemeinen gibt man 0,05 bis 0,5, vorzugsweise 0,05 bis 0,1 Gew.-% an festem alkalischem Mittel, bezogen auf die Mischung aus Ausgangsmethanol und zurückgeführtem wäßrigem Methanol, zu.

Der Endstoff wird in Gestalt eines Teiles der Absorptionslösung der Stufe 1 entnommen. Er enthält von 18 bis 30, vorzugsweise von 19 bis 25 Gew.-% Gesamtharnstoff, von 35 bis 45, insbesondere von 38 bis 43 Gew.-% Gesamtformaldehyd in Form von 16 bis 20, vorzugsweise von 17,5 bis 19,4 Gew.-% freiem Formaldehyd, von 17 bis 21, vorzugsweise von 18 bis 20 Gew.-% Methylolharnstoffen, von 1,6 bis 2,8, vorzugsweise von 2,1 bis 2,6 Gew.-% Mono- und Polymethylenharnstoffen, von 0,29 bis 0,7, vorzugsweise von 0,3 bis 0,6 Gew.-% Polymethylolverbindungen von Methylenharnstoffen, von 0,1 bis 0,3, vorzugsweise von 0,15 bis 0,25 Gew.-% Methylenetherverbindungen, von 0,01 bis 0,2, vorzugsweise von 0,01 bis 0,15 Gew.-% höhermolekularen Kondensaten, von 0,2 bis 3, vorzugsweise von 0,4 bis 1,6 Gew.-% Methanol, von 0,005 bis 0,05, vorzugsweise von 0,006 bis 0,03 Gew.-% Nebenstoffe (z. B. Ameisensäure).

Gewünschtenfalls kann der Endstoff für eine Luftwäsche analog der DE-PS 2 451 990 verwendet werden. Die Behandlung selbst erfolgt zweckmäßig in Form einer Gaswäsche. Luft und gegebenenfalls Inertgas wird in einem Verhältnis von 20 bis 80, vorzugsweise von 30 bis 50 Volumenteilen Gas je Volumenteil Lösung von Formaldehyd und Harnstoff als Waschlösung gewaschen. Die Wäsche wird in der Regel in Waschtürmen (Waschkolonnen) durchgeführt, z. B. wird die Waschflüssigkeit am Kopf oder in der oberen Hälfte der Kolonne zugegeben und die Reaktionsluft zweckmäßig im Gegenstrom von der Kolonnenblase her nach oben durch die Kolonne geleitet. Die Wäsche wird in der Regel bei einer Temperatur von 50 bis 100, vorzugsweise von 70 bis 85°C (gemessen an der Eintrittstelle der Waschlösung), drucklos oder unter Druck, diskontinuierlich oder in der Regel kontinuierlich durchgeführt. Die Luft wird nach der Wäsche vorteilhaft dem Methanol/Wasser-Verdampfer zweckmäßig oberhalb des Verdampfersumpfes und der Methanolzuführung zugeführt und mit dem Dampfgemisch des Verdampfers vermischt. Der Luftwäsche wird dann der Endstoff in Gestalt seiner Lösung entnommen. In einer vorteilhaften Ausführungsform werden von der Waschlösung nach der Wäsche der 10. bis 2. Teil der weiteren Verwendung der Lösung (Lösung des Endstoffs), z. B. der Leimherstellung zugeführt und der Rest zum Kopf der Waschkolonne zurückgeführt.

Der nach dem Verfahren der Erfindung herstellbare Formaldehyd ist in Gestalt seiner wäßrigen Absorptionslösung mit Harnstoff wertvoller Ausgangsstoff für die Herstellung von Kondensationsharzen in Form von Holzleimen und Tränkharzen für die Spanplattenindustrie und von Hilfsmitteln für die Textil-, Papier- und Lackindustrie. Bezüglich der Verwendung wird auf Ullmanns Encyklopädie der technischen Chemie, Band 7, Seite 403—424, verwiesen.

Die Figur veranschaulicht eine der vorgenannten bevorzugten Ausführungsformen, die gleichzeitig auch für die Beispiele Verwendung findet.

## Beispiel 1

220,3 kg Luft wurden stündlich durch Leitung (1) dem Verdampfer (4) am unteren Ende zugeführt. Gleichzeitig gelangten stündlich 118 kg frisches Methanol durch die Leitung (2), 55,7 kg Kondensat aus Leitung (33) und 21,1 kg Wasser durch die Leitung (3) in den Verdampfer (4). Das Dampfgemisch verließ den Verdampfer (4) und gelangte über die Leitung (5) in den Reaktor (6). Aus diesem wurde stündlich ein Reaktionsgemisch von 100 kg Gesamtformaldehyd, 129,4 kg Wasser, 4,9 kg Methanol und 183 cbm Inertgas durch Leitung (7) abgezogen und auf den Kopf der 1. Absorptionskolonne (8) geleitet.

Im Gleichstrom mit einem Anteil (stündlich 276,4 kg) an Sumpf (stündlich 83,5 kg Gesamtharnstoff, 81,8 kg Gesamtformaldehyd und 2,4 kg Methanol) der zweiten Absorptionskolonne (10), der durch Leitung (9) zugeführt wurde, durchzog das Dampf/Gasgemisch die Kolonne (8) (1. Absorptionsstufe) und über Leitung (34) den unteren (Stufe 2) und oberen Teil (Stufe 3) der Kolonne (10). Es bildete sich am Kolonnenboden der Kolonne (8) ein Sumpf der 3 Stufen, der auf konstanter Höhe gehalten wird. Er wies eine Temperatur von 75°C auf. Mittels Pumpe (11) wurden stündlich 167,5 kg über die Leitung (12) auf die Mitte der 2. Absorptionskolonne (= Kopf von Stufe 2) sowie 250 kg über die Leitung (13) als Endstoff abgeleitet. Der größte Teil (stündlich 5018 kg) des Sumpfes (stündlich 2007,2 kg Gesamtformaldehyd, 1003,6 kg Gesamtharnstoff und 64,2 kg Methanol) gelangte über Leitung (14) in die Kolonne

(8) zurück. Die Verweilzeit betrug in der 1. Stufe 86 Minuten, das Molverhältnis 4 Mol Formaldehyd je Mol Harnstoff.

Durch Leitung (15) wurden stündlich am Kopf des oberen Teiles der Absorptionskolonne (10) (3. Absorptionsstufe) 50 kg Harnstoff als 70gewichtsprozentige, wäßrige Harnstofflösung zugeführt. Die Lösung wurde im Gegenstrom zu dem aus dem unteren Teil entweichenden und über Boden (16) in den oberen Teil der Kolonne (10) gelangten gasförmigen Reaktionsgemisch geführt. Ein Teil des Sumpfes (80° C) dieser Kolonne wurde über Pumpe (17) und Leitung (18) im Kreis zurückgeführt. Die Verweilzeit betrug in der 3. Stufe 48 Minuten, das Molverhältnis 0,16 Mol Formaldehyd je Mol Harnstoff.

Aus dem Rückführstrom wurden durch Leitung (19) stündlich 88,3 kg Lösung (50 kg Gesamtharnstoff, 4,0 kg Gesamtformaldehyd und 0,3 kg Methanol) abgezogen, mit einem Anteil des Sumpfes der 1. Absorptionsstufe (stündlich 167,5 kg Lösung) mit 67 kg Gesamtformaldehyd und 33,5 kg Gesamtharnstoff aus Leitung (12) vermischt und dann binnen 2 Sekunden auf den Kopf (20) des unteren Teiles (2. Absorptionsstufe) der Kolonne (10) geleitet. Stündlich gelangten 255,8 kg Lösung (71 kg Gesamtformaldehyd, 83,5 kg Gesamtharnstoff, 2,4 kg Methanol) mit einem mittleren Molverhältnis von 1,70 Molen Formaldehyd pro Mol Harnstoff durch Leitung (21) auf den Kopf der 2. Absorptionsstufe, trafen im Gegenstrom auf das gasförmige Reaktionsgemisch und bildeten am Boden der Kolonne (10) den Sumpf der 2. Absorptionsstufe. Er besaß eine Temperatur von 74° C, die Verweilzeit betrug in der 2. Stufe 22 Minuten, das Molverhältnis 2,0 Mol Formaldehyd je Mol Harnstoff.

3517 kg Sumpf im unteren Teil der Kolonne (10) wurden stündlich im Kreislauf über Pumpe (22) und Leitung (23) zum Kopf (20) des unteren Teiles (2. Absorptionsstufe) der Kolonne (10) geführt, wobei stündlich 835,2 kg dieser Lösung (247,2 kg Gesamtformaldehyd, 252,3 kg Gesamtharnstoff und 7,2 kg Methanol) auf dem Umweg über Leitung (24) in das Mischgefäß (25) (77° C) geleitet wurden, in dem noch über Ameisensäurezuleitung (26) ein pH von 5,8 eingestellt wurde und in dem nach einer Verweilzeit von 30 Minuten ein Reaktionsgemisch erhalten wurde, das über Pumpe (27) und Leitung (28) mit dem Hauptstrom des Sumpfes aus Leitung (23) vereinigt wurde.

Am Kopf der Kolonne (10) wurde das Abgas über Leitung (29) durch den Kühler (30) geführt. Die flüssigen Anteile gelangten in den Abscheider (31). Aus Leitung (32) entwich das Abgas. Aus dem Abscheider (31) wurde das Kondensat, das aus stündlich 1,7 kg Methanol und 54 kg Wasser bestand, über Leitung (33) zusammen mit Frischmethanol dem Verdampfer (4) zugeleitet.

Die Formaldehydausbeute betrug stündlich 100 kg (90,4% der Theorie). Der pH betrug im Sumpf der Kolonne (8) 6,2, der Kolonne (10) (unterer Teil) 7,1, der Kolonne (10) (oberer Teil) 8,2. Der Gesamtformaldehyd-Gehalt der Sumpflösungen der 3 Stufen betrug 1) 40; 2) 29,6; 3) 4,5 Gewichtsprozent. Die als Endstoff anfallende wäßrige Formaldehyd-Harnstoff-Lösung wurde über Leitung (13) entnommen und enthielt 40 Gew.-% Gesamtformaldehyd, 17,9 Gew.-% freien Formaldehyd, 20 Gew.-% Gesamtharnstoff, 18,7 Gew.-% Formaldehyd in Form von Mono- und Polymethylolharnstoffen, 2,6 Gew.-% als Mono- und Polymethylenharnstoffe, 0,5 Gew.-% als Polymethylolverbindungen von Methylenharnstoffen, 0,2 Gew.-% als Methylenetherverbindungen, 0,1 Gew.-% als höhermolekulare Kondensate, 1,3 Gew.-% Methanol, 0,008 Gew.-% Nebenstoffe (z. B. Ameisensäure). Das Molverhältnis betrug 4,0 Mol Formaldehyd je Mol Harnstoff.

Beispiel 2

Die Absorption wurde analog Beispiel 1 durchgeführt.

220,3 kg Luft wurden stündlich einer Waschkolonne am unteren Ende zugeführt. Im Gegenstrom hierzu wurden stündlich 260,2 kg der Lösung des Endstoffs der Zusammensetzung von Beispiel 1 aus Leitung (13) der Waschkolonne zugeführt, wobei sich die Luft stündlich mit 1,2 kg Methanol, 56,3 kg Wasser und 6,5 kg Gesamtformaldehyd belud. Die Luft wurde dann über Leitung (1) in das dampfförmige Methanol-Wasser-Gemisch in Leitung (5) eingeleitet. Die Waschlösung wurde aus dem Sumpf der Waschkolonne im Kreislauf zum Kopf der Waschkolonne geführt, wobei stündlich 200 kg Lösung des Endstoffs dem Kreislauf entnommen und der weiteren Verwendung zugeführt wurde.

Die als Endstoff anfallende Formaldehyd-Harnstoff-Lösung hatte die folgende Zusammensetzung: 50 Gew.-% Gesamtformaldehyd, 22,4 Gew.-% freien Formaldehyd, 25 Gew.-% Gesamtharnstoff, 23,4 Gew.-% Mono- und Polymethylolharnstoffe, 3,2 Gew.-% Mono- und Polymethylenharnstoffe, 0,6 Gew.-% Polymethylolverbindungen von Methylenharnstoffen, 0,3 Gew.-% Methylenetherverbindungen, 0,1 Gew.-% höhermolekulare Kondensate, 0,3 Gew.-% Methanol, 0,008 Gew.-% Nebenstoffe (z. B. Ameisensäure).

**Patentanspruch**

Verfahren zur Herstellung von konzentrierten, wäßrigen Lösungen von Formaldehyd und Harnstoff durch oxidierende Dehydrierung von Methanol mit Luft in Gegenwart eines Silberkatalysators bei erhöhter Temperatur und anschließender Absorption des so gebildeten Formaldehyds in einer wäßrigen Lösung von Formaldehyd und Harnstoff, dadurch gekennzeichnet, daß die Absorption in einer ersten Stufe in einer wäßrigen Lösung von Formaldehyd und Harnstoff mit einem Molverhältnis von 2 bis 5 Mol Formaldehyd je 1 Mol Harnstoff bei einem pH von 4 bis 7, dann in einer 2. Stufe mit einer wäßrigen Lösung von Formaldehyd und Harnstoff in einem Molverhältnis von 1,8 bis 2,6 Mol Formaldehyd je Mol Harnstoff bei einem pH von 6,5 bis 8 und in einer 3. Stufe in einer wäßrigen Lösung von Formaldehyd und Harnstoff mit einem Molverhältnis von 0,03 bis 0,5 Mol Formaldehyd je 1 Mol Harnstoff bei einem pH von 8 bis 9 durchgeführt wird, wobei gleichzeitig ein Anteil der Lösung aus der 1. Stufe mit einem Anteil der Lösung aus der 3. Stufe miteinander so gemischt werden, daß sich in dem gebildeten Gemisch ein Molverhältnis kleiner als 1,8 Mol Formaldehyd je Mol Harnstoff einstellt, und das Gemisch der Lösung der Stufe 2 zugeführt und ein weiterer Anteil der Lösung aus Stufe 2 der Stufe 1 zugeführt und ein weiterer Anteil der Lösung aus Stufe 2 mit Säure bei pH 4 bis 6,5 behandelt wird und in die Stufe 2 zurückkehrt, und man gewünschtenfalls die aus der 1. Stufe abgezogene Lösung des Endstoffs einer Luftwäsche unterzieht.

**Claim**

A process for the production of concentrated, aqueous solutions of formaldehyde and urea by oxidizing dehydrogenation of methanol with air in the presence of a silver catalyst at elevated temperature, and subsequent absorption of the formaldehyde thus formed in an aqueous solution of formaldehyde and urea, wherein the absorption is carried out in a first stage in an aqueous solution of formaldehyde and urea in a molar ratio of 2 to 5 : 1 at a pH of 4 to 7, then in a second stage in an aqueous solution of formaldehyde and urea in a molar ratio of 1.8 to 2.6 : 1 at a pH of 6.5 to 8, and in a third stage in an aqueous solution of formaldehyde and urea in a molar ratio of 0.03 to 0.5 : 1 at a pH of 8 to 9, a portion of the solution from the first stage being simultaneously mixed with a portion of the solution form the third stage in such a manner that the molar ratio of formaldehyde to urea in the resulting mixture is $<1.8 : 1$, which mixture is fed to the solution of the second stage, and a further portion of the solution from the second stage being fed to the first stage, and a further portion of the solution from the second stage being treated with acid at a pH of 4 to 6.5 and returned to the second stage, and, if desired, the solution of the end product withdrawn from the first stage is scrubbed with air.

**Revendication**

Procédé de préparation de solutions aqueuses concentrées de formaldéhyde et d'urée par une déshydrogénation oxydante du méthanol par l'air à température accrue en présence d'un catalyseur à l'argent, suivie de l'absorption du formaldéhyde obtenu dans une solution aqueuse de formaldéhyde et d'urée, caractérisé en ce que l'absorption est réalisée dans un premier stade dans une solution aqueuse de formaldéhyde et d'urée avec un rapport molaire de 2 à 5 moles de formaldéhyde pour 1 mole d'urée, d'un pH de 4 à 7, dans un deuxième stade dans une solution aqueuse de formaldéhyde et d'urée avec un rapport molaire de 1,8 à 2,6 moles de formaldéhyde pour 1 mole d'urée, d'un pH de 6,5 à 8, et dans un troisième stade dans une solution aqueuse de formaldéhyde et d'urée avec un rapport molaire de 0,03 à 0,5 mole de formaldéhyde pour 1 mole d'urée, d'un pH de 8 à 9, avec mélange simultané d'une fraction de la solution du premier stade et d'une fraction de la solution du troisième stade en des proportions telles que, dans le mélange formé, le rapport molaire est inférieur à 1,8 mole de formaldéhyde pour 1 mole d'urée, ce mélange étant introduit dans la solution du stade 2, recyclage d'une autre fraction de la solution du stade 2 dans le stade 1 et traitement d'une autre fraction de la solution du stade 2 par un acide à un pH de 4 à 6,5 et retour dans le stade 2, la solution du produit final, soutirée du premier stade, étant éventuellement soumise à un lavage à l'air.

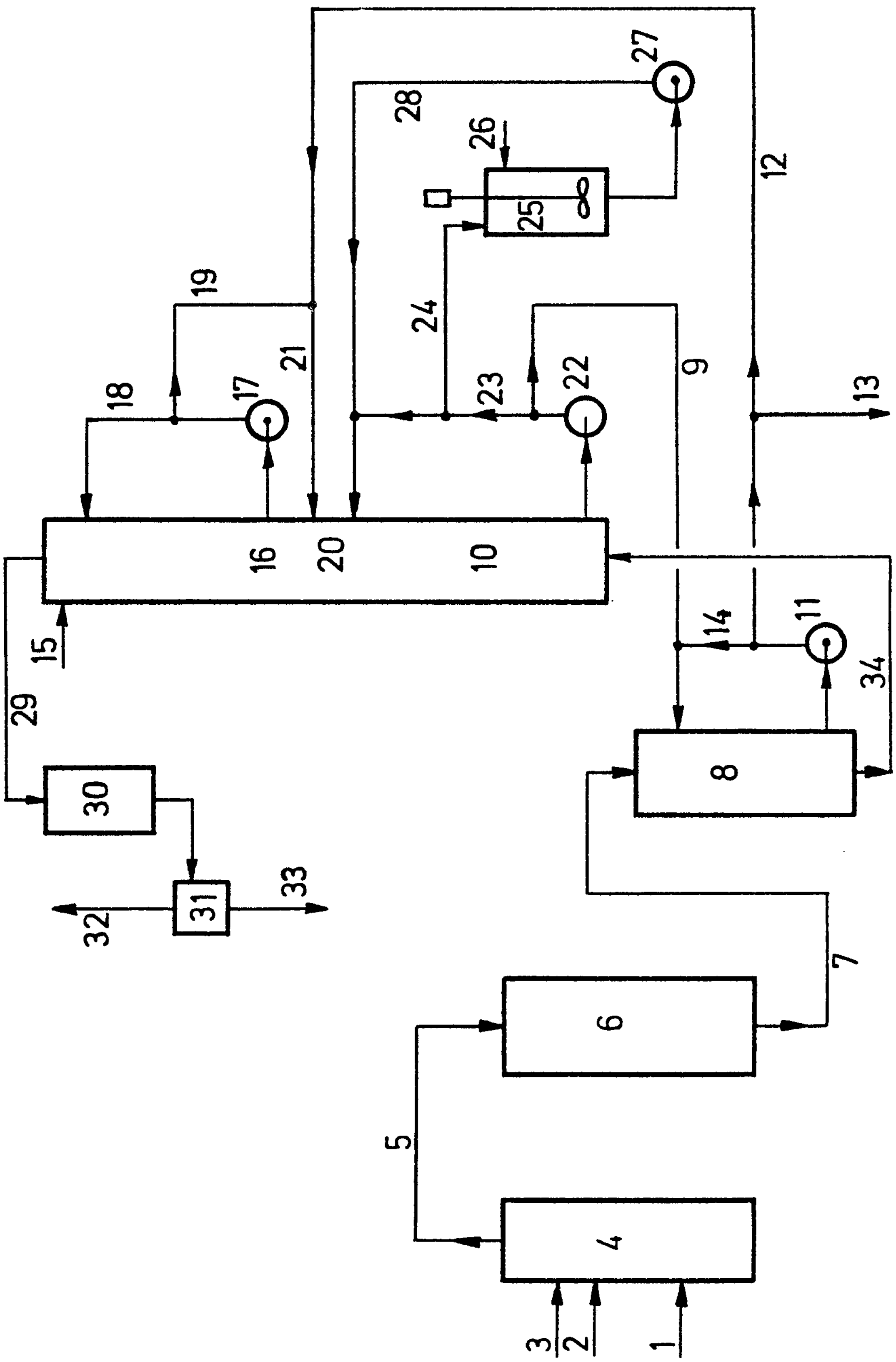
1
2
3
4
5
6
7
8
9
10
11
12
13
14
15
16
17
18
19
20
21
22
23
24
25
26
27
28
29
30
31
32
33
34